# EUROPEAN PATENT SPECIFICATION

(11) **EP 4 370 931 B1**
(45) Date of publication and mention of the grant of the patent: **03.09.2025**
(21) Application number: 22748346.8
(22) Date of filing: 12.07.2022
(51) Int. Cl.: G01N 33/74, G01N 33/92

(54) **BIOMARKERS FOR ALZHEIMER'S DISEASE**
BIOMARKER FÜR MORBUS ALZHEIMER
MARQUEURS BIOLOGIQUES DE LA MALADIE D'ALZHEIMER

(30) Priority: 13.07.2021 IT 202100018455
(43) Date of publication of application: 22.05.2024
(73) Proprietor: Fondazione Istituto Italiano di Tecnologia, 16163 Genova (GE) (IT); Fondazione Santa Lucia, 00179 Roma (IT)
(72) Inventor: ARMIROTTI, Andrea, 16138 GENOVA (GE) (IT); OTTONELLO, Giuliana, 16145 GENOVA (GE) (IT); FRANCESCHI, Pietro, 38098 SAN MICHELE ALL'ADIGE (TN) (IT); SCHMIDT, Reinhold, 8036 GRAZ (AT); SPALLETTA, Gianfranco, 00147 ROMA (RM) (IT)
(74) Representative: Bianchetti & Minoja with Trevisan & Cuonzo IPS SRL
(86) International application number: PCT/EP2022/069466
(87) International publication number: WO 2023/285462

(56) References cited:
- WO-A1-2019/091952
- PALESE FRANCESCA: "Neuroprotective role of N-acylphosphatidylethanolamines in Parkinson's disease", 1 January 2017 (2017-01-01), pages 1 - 65, XP055898159, Retrieved from the Internet <URL:http://amsdottorato.unibo.it/7939/1/PhD%20thesis%20Francesca%20Palese.pdf> [retrieved on 20220307]
- HAMID ZEESHAN ET AL: "Gender specific decrease of a set of circulating N-acylphosphatidyl ethanolamines (NAPEs) in the plasma of Parkinson's disease patients", METABOLOMICS, SPRINGER US, NEW YORK, vol. 15, no. 5, 3 May 2019 (2019-05-03), pages 1 - 9, XP036773721, ISSN: 1573-3882, [retrieved on 20190503], DOI: 10.1007/S11306-019-1536-Z
- ASTARITA GIUSEPPE ET AL: "Identification of biosynthetic precursors for the endocannabinoid anandamide in the rat brain", vol. 49, no. 1, 1 January 2008 (2008-01-01), US, pages 48 - 57, XP055898190, ISSN: 0022-2275, Retrieved from the Internet <URL:https://reader.elsevier.com/reader/sd/pii/S0022227520428792?token=4E77A1FF3217F3D8514F0E771173E3BF023EF43552C7D54C08BD0EE0AFDA1F468F02420C21BEDF4CDD04EEDB2B53F25B&originRegion=eu-west-1&originCreation=20220307125924> DOI: 10.1194/jlr.M700354-JLR200
- MARK MAPSTONE ET AL: "Plasma phospholipids identify antecedent memory impairment in older adults", NATURE MEDICINE, vol. 20, no. 4, 1 January 2014 (2014-01-01), New York, pages 415 - 418, XP055351904, ISSN: 1078-8956, DOI: 10.1038/nm.3466

## Description

The present invention relates to a method for the diagnosis or prognosis of Alzheimer's disease, or prevention of its onset, based on analysis of N-acylphosphatidylethanolamine (NAPE) molecules as biomarkers. The invention also provides a kit for implementation of the method for diagnosis, prognosis or prediction of the disease, comprising means and reagents which are useful to determine the concentration of the biomarkers in the test sample.

### Background of the invention

Alzheimer's disease (AD) is a long-term neurodegenerative disorder that affects the motor and cognitive functions, language, character and mental activity of individuals suffering from it. With the progressive aging of the population, the incidence of AD is constantly increasing, and represents a huge challenge for the national health system. On the basis of current data, it has been estimated that in the European countries, 9 out of 1000 people between 75 and 79 years of age may develop AD every year. At the present time, the diagnosis and prognosis of AD are essentially based on clinical assessment, cerebrospinal fluid imaging and/or radiological and nuclear medicine (mainly magnetic resonance and positron-emission tomography). However, none of these approaches is sensitive enough to provide a definite early diagnosis, because the symptoms of brain damage must be well-established before making a final diagnosis *in vivo.* In fact, the disease can only be diagnosed when clinical symptoms are already detectable, i.e.. when the neuronal damage is at an advanced stage and the therapeutic options available to doctors (especially neurologists, psychiatrists and geriatricians) are greatly reduced. At present, no effective molecular biomarkers for AD are available in clinical practice, although several have been proposed. It would therefore be highly desirable to have a biomarker measurable with a simple blood test, which is predictive of the disease or correlated with its onset and progress, and enables its development to be evaluated over time.

### State of the art

WO2019/091952, filed by the present applicants, describes a method for the diagnosis or prognosis of neurodegenerative disease comprising analysis of the plasma levels of various N-acylphosphatidylethanolamines (NAPEs), considered individually and/or combined with one another. The authors observed a correlation between a reduction in plasma concentration of NAPE molecules and the onset on progression of Parkinson's and Alzheimer's diseases, and identified the threshold values of the biomarkers that discriminate between individuals suffering and not suffering from said diseases.

Similar results, relating to biomarkers for Parkinson's disease, are reported in the article by Hamid Z. et al., "Gender specific decrease of a set of circulating N-acylphosphatidylethanolamines (NAPEs) in the plasma of Parkinson's disease patients", Metabolomics (2019) 15:74.

WO2007/050318 describes a method of correlating the lipid profile with the progression of a central nervous system (CNS) disorder, such as a psychiatric or neurodegenerative disorder. The metabolites cited as biomarkers for CNS disorders include phosphatidylethanolamines and phosphatidylcholine.

EP1482920 relates to the use of phospholipid mixtures containing N-acetylphosphatidylethanolamines (NAPEs) and/or phospholipid mixtures containing N-acetylethanolamines (NAEs) together with phosphatidic acids (PAs) and/or lysophosphatidic acids (LPAs) for the preparation of medicaments having anorexic activity or of medicaments or foodstuffs for the treatment of various disorders, such as AD, PD and senile dementia.

The article by Coulon, D. et al., "Occurrence, biosynthesis and functions of N-acylphosphatidylethanolamines (NAPE): Not just precursors of N-acylethanolamines (NAE)", describes the role of bioactive N-acylphosphatidylethanolamine molecules in various physiological processes. The article shows that NAPE levels increase strongly in response to stress conditions involving degenerative changes of the cell plasma membrane. In particular, NAPE levels are greatly increased in the cortex, hippocampus and inferior colliculus.

EP2950102 describes a method for establishing the probability with which a patient with a cognitive disorder may develop AD. The method is based on detection of the levels of various biomarkers, including ceramide, in blood, serum or plasma samples.

### Description of the invention

It has now been discovered that certain NAPEs naturally present in human plasma, other than those previously described by the same inventors in WO2019/091952, discriminate between individuals suffering and not suffering from Alzheimer's disease (AD) with a greater level of accuracy than the group of NAPE molecules described in the prior patent application, and therefore represent an effective biomarker for AD.

To arrive at this result, plasma samples from AD patients were analyzed by comparing them with those of the same number of cognitively healthy individuals who were comparable with the AD patients on the basis of gender and age. The plasma samples were analyzed to determine the level of the various NAPE species, using a liquid chromatography method combined with mass spectrometry (LC-MS/MS).

The samples were acquired in random order from a set of different batches, including quality control samples (murine plasma mixture). The data were grouped and analyzed by multivariate analysis techniques, as a result of which a group of NAPEs able to differentiate between sick and healthy subjects was identified. The discriminating efficacy of the NAPEs was then confirmed by further statistical analyses, and a correlation pattern was defined for each molecule, on the basis of its variation from the controls, consisting of an increase ("upregulation") or decrease ("downregulation") in NAPE concentration. On the basis of a comparison between the Receiver Operating Characteristic (ROC) curves of the NAPE molecules according to the present invention and those described in WO2019/091952, the former proved significantly more effective as biomarkers and/or predictors of AD.

Analysis of the distribution of the amounts of the various NAPEs in the healthy population led to identification of reference plasma values for each of said NAPEs. Values measured outside that range are indicative of the presence of the disease, its progression, or the risk of its onset. Values significantly different from the reference values may require further diagnostic investigations to establish the presence or progression of the disease, for example by means of more detailed cognitive tests.

Therefore, according to a first aspect thereof, the invention provides an *in vitro* method for determining the presence or risk of onset of Alzheimer's disease in an individual, monitoring its development over time or determining the efficacy of a pharmacological therapy designed for its treatment, wherein said method comprises:
a) determining, in a plasma sample of an individual, the concentration of each of
the following N-acylphosphatidylethanolamines (NAPEs):

| | | |
|---|---|---|
| P16:0/18:1/N16:0; | P18:0/18:2/N16:0; | P16:0/22:5/N16:0; |
| P16:0/22:4/N16:0; | P16:0/22:5/N18:1; | P18:0/20:4/N18:1; |
| P18:0/20:5/N18:0; | P18:0/22:4/N16:0; | P16:0/22:4/N18:0; |
| P18:0/22:6/N18:1; | P18:0/22:4/N18:1; | and P18:0/22:4/N18:0; |

b) comparing the concentration of each NAPE with a reference value corresponding to the concentration of the same NAPE in the plasma of individuals not suffering from Alzheimer's disease, wherein a difference between each NAPE concentration found in the plasma sample of the test subject and the corresponding reference value indicates the presence or risk of onset of Alzheimer's disease, its progression, or the inefficacy of the pharmacological treatment.

The NAPE structures reported herein refer to the fatty acid chains of c carbon atoms and n double bonds bonded in the Sn1 and Sn2 positions of glycerol, and to the nitrogen atom of the ethanolamine residue, according to the scheme *cc:n*/*cc:n*/N*cc:n.* The fatty acid bonded in the Sn1 position is always a plasmalogen (ether lipid). For example, NAPE P16:0/18:1/N16:0 therefore has this structure:

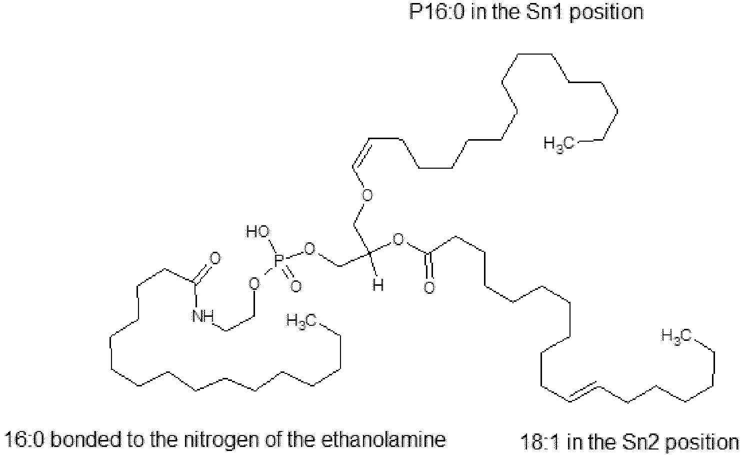

The plasma concentration of NAPE is preferably determined by liquid chromatography analysis with tandem mass spectrometry (LC-MS/MS), as described in Hamid Z. et al., "Gender specific decrease of a set of circulating N-acylphosphatidylethanolamines (NAPEs) in the plasma of Parkinson's disease patients", Metabolomics (2019) 15:74, or by the procedure described in Basit A. et al., "Ion mobility mass spectrometry enhances low-abundance species detection in untargeted lipidomics", Metabolomics (2016) 12:50, both of which are incorporated herein for reference.

In one embodiment, the LC-MS/MS method is performed with an automated instrument able to analyze different samples arranged on multiwell plates.
Preferably, the NAPE species present in the plasma sample are quantitated using a calibration curve prepared by adding a synthetic NAPE at increasing concentrations to commercial human plasma.

The difference in concentration compared with the reference value can consist of an increase or decrease in the plasma concentration of a given NAPE, depending on whether the latter is up- or downregulated. In particular, said difference corresponds to an increase for the following species: P16:0/18:1/N16:0; P16:0/22:5/N16:0; P16:0/22:4/N16:0; P16:0/22:5/N18:1; P18:0/20:4/N18:1; P18:0/22:6/N18:1; and a decrease for the following species: P18:0/18:2/N16:0; P18:0/20:5/N18:0; P18:0/22:4/N16:0; P16:0/22:4/N18:0 P18:0/22:4/N18:1; P18:0/22:4/N18:0.

In a preferred embodiment, the increase or decrease compared with the reference value is (in modulus) greater than 5%, preferably 10%, more preferably 30%, yet more preferably greater than 60%.

Univariate statistical analysis of the data acquired from analysis of the population not suffering from AD led to determination of the following reference values for each NAPE:

| **NAPE** | **reference value (pmol/ml)** |
|---|---|
| P16:0/18:1/N16:0 | 30.1 |
| P18:0/18:2/N16:0 | 40.4 |
| P16:0/22:5/N16:0 | 84.4 |
| P16:0/22:4/N16:0 | 190.0 |
| P16:0/22:5/N18:1 | 33.2 |
| P18:0/20:4/N18:1 | 74.9 |
| P18:0/20:5/N18:0 | 31.6 |
| P18:0/22:4/N16:0 | 21.8 |
| P16:0/22:4/N18:0 | 49.9 |
| P18:0/22:6/N18:1 | 19.2 |
| P18:0/22:4/N18:1 | 7.5 |
| P18:0/22:4/N18:0 | 7.1 |

The values indicated above, though significant, reliable and applicable to large-scale screening, relate to the interval between the 5th and 95th quantiles of distribution of the plasma NAPE values observed in the cognitively healthy control population. Said values may vary according to the population sample examined (age, geographical origin) and its number. In any event the skilled person, on the basis of common general knowledge of the field, is able to determine the reference values for a given population.

For a given combination of multiple NAPE species, the higher the number of NAPEs that meet the variation criteria compared with the corresponding reference values as specified above, the greater the predictive value of the test. The maximum predictive value of the method occurs when all 12 of the NAPEs analyzed simultaneously meet the specified variation criteria.

A further aspect of the invention relates to a kit for implementation of the method described herein, comprising, as internal standards, known amounts of the NAPEs herein disclosed and, in separate containers, reagents and buffers for preparation of the sample to be analyzed or analysis of the NAPE molecules, in particular using the LC-MS/MS technique.

The examples below and the annexed figures illustrate the invention in greater detail.

### Brief description of figures

**Figure 1****:** diagram of score obtained from PCA (Principal Component Analysis) for the dataset of AD patients.
**Figure 2****:** VIP diagram for the dataset of AD patients. The number relates to the 12 NAPE species listed in Table 1. The model includes the "age" variable together with the biomarkers.
**Figure 3****:** Superimposition of the ROC curves relating to (i) the best markers resulting from the preceding study (Hamid Z et al., Metabolomics 2019 - cohort of 30 AD patients and 30 controls), represented by the top curve, and (ii) the best markers obtained from the data obtained in the present study (65 AD patients vs 65 controls), represented by the bottom curve.

### Examples

### Example 1

### Analysis and determination of plasma levels of the 12 NAPE species

The plasma samples, stored at -80°C until analysis, are gradually thawed to 4°C. 0.2 ml aliquots of plasma are then dispensed into a 96-well plate comprising wells with a volume of 2 ml. The pNAPEs are then extracted by adding 1 ml of isopropanol to each well; the solution is then stirred on a vortex for 1 minute, and then centrifuged at 3000 rpm, 4°C, for 15 minutes. 0.9 ml of supernatant is then collected and transferred to a 96-well plate comprising 1 ml deep wells, wherein they are then dried under nitrogen current. The entire procedure for extracting the NAPEs from the plasma samples is conducted with an automated workstation for preparation of the sample, to optimize the reproducibility of the protocol and the efficiency of extraction. At the time of analysis, the samples are then resuspended in 0.07 ml of solution 9: 1 MeOH: CHCl3 for LC-MS/MS analysis. The targeted NAPE analysis is then conducted on a UPLC Waters Acquity system coupled with a Xevo TQ-MS triple-quadrupole mass spectrometer. Similar triple-quadrupole systems made by other manufacturers (Agilent, Sciex, Shimadzu and Thermo) will give similar results to those described in the present application after a suitable method transfer stage. The NAPEs are separated chromatographically on a BEH C18 reverse-phase column (2.1 x 50 mm, 1.7 um), the temperature of which is maintained at 55°C, and eluted at a flow rate of 0.450 ml /min using the following gradient conditions: A = 10 mM ammonium formate in acetonitrile / water (60:40 v/v), B = 10 mM ammonium formate in isopropanol / acetonitrile (90:10 v/v). The following gradients are applied to the chromatography column:
0.0-1.0 min 40% B,
1.0-7.0 min 40-100% B
7.0-8.0 min at 100% B.

The column is then reconditioned to 40% B for 1 min. The total analysis time is 9 min, and the injection volume is set to 5 µl. The mass spectrometer operates in positive ESI mode, and the analytes are quantified by multiple reaction monitoring (MRM). The voltages of the capillary tube and sampling cone were set to 3 kV and 30 V respectively for all transitions. The source temperature was set to 120°C. The desolvation and gas flows of the cone (N2) were set to 800 and 50 l/h respectively. The desolvation temperature was set to 450°C. The analyses are conducted by randomizing the samples and acquiring them in several batches. Each batch must include at least one QC sample (consisting of murine plasma) every ten samples, which included a number of quality control samples. The values measured in the quality controls serve to realign the results of the various batches. The NAPE species present in the plasma are quantitated using a 6-point calibration curve prepared by adding a synthetic NAPE (18:1/18:1/N19:0) at increasing concentrations (1 to 500 nM) to commercial human plasma. The instrumental response obtained for the synthetic NAPE can be deemed similar to that of the endogenous NAPEs. This allows the area under each chromatographic peak to be converted to the corresponding concentration value, expressed in picomol / ml.

**Table 1 below shows, for each NAPE, the MS/MS parameters used for their detection and quantitation:**

| **ID** | **Name** | **Parent m/z** | **Fragment m/z** |
|---|---|---|---|
| 1 | P16:0/18:1/N16:0 | 940.8 | 282.3 |
| 2 | P18:0/18:2/N16:0 | 966.8 | 282.3 |
| 4 | P16:0/22:5/N16:0 | 988.8 | 282.3 |
| 5 | P16:0/22:4/N16:0 | 990.8 | 282.3 |
| 7 | P16:0/22:5/N18:1 | 1014.8 | 308.3 |
| 10 | P18:0/20:4/N18:1 | 1016.8 | 308.3 |
| 11 | P18:0/20:5/N18:0 | 1016.8 | 310.3 |
| 12 | P18:0/22:4/N16:0 | 1018.8 | 282.3 |
| 13 | P16:0/22:4/N18:0 | 1018.8 | 310.3 |
| 15 | P18:0/22:6/N18:1 | 1040.8 | 308.3 |
| 17 | P18:0/22:4/N18:1 | 1044.8 | 308.3 |
| 18 | P18:0/22:4/N18:0 | 1046.8 | 310.3 |

### Example 2

### Data analysis

Plasma samples of AD patients (65, 33M/32F, aged 50 to 91 years) were analyzed comparatively with a control group of cognitively healthy individuals (65, 34M/31F, aged 54 to 81). The plasma NAPE levels were measured with the LC-MS/MS method described in Example 1. The samples were acquired in random order from a set of different batches. QC samples (mouse plasma, 6 samples per batch) were included in the list. After correction for the batch effect, conducted by realigning the values observed in the QC samples, taken as invariant, all the data were combined and analyzed by multivariate analysis techniques.

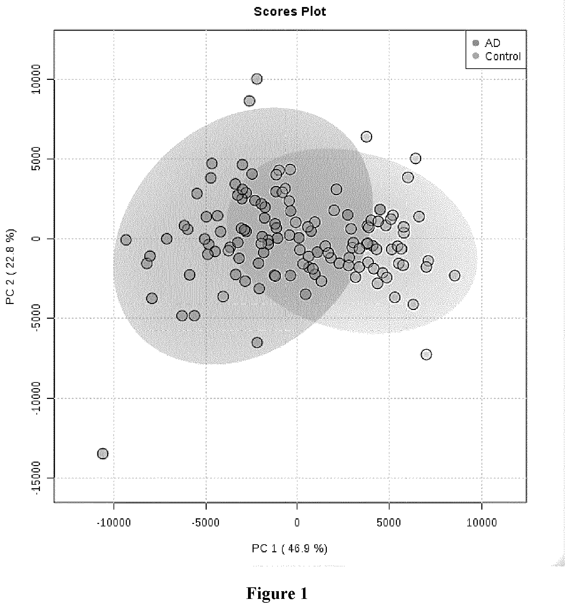

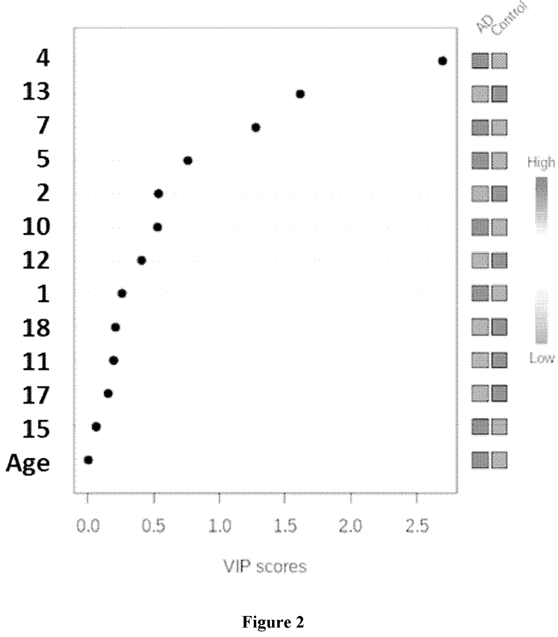

Figure 1 shows the diagram of the score obtained from PCA (Principal Ingredient Analysis) of the dataset. The diagram clearly demonstrates that the NAPE group analysis differentiates between AD patients and healthy individuals. A subsequent supervised analysis of the importance of each lipid compared with the discriminant power of the model demonstrates that all 12 NAPEs selected help to predict the patients' pathological state to a greater extent than the age variable (Figure 2). The trend of said biomarkers differs in the AD and PD populations: in Parkinson's disease (PD), a general reduction in the 5 NAPEs was observed, whereas in the AD population, some NAPEs were downregulated (pale squares), while others were upregulated (dark squares).

The molecules analyzed exhibit a complex system of reciprocal correlations, which contributes to the high predictive value of the NAPEs selected.

Univariate statistical analysis was then conducted on the novel group of biomarkers, to calculate the threshold of change in circulating levels of NAPEs, which is useful for diagnostic purposes. Table 2 summarizes the results and displays the mean difference in the threshold values observed in the AD population compared with the controls, the significance (P values in a comparative unpaired t-test), and the values of the area under the curve (AUC) of the corresponding Receiver Operating Characteristic (ROC) curve.

**Table 2: differences observed in the plasma concentration of the 12 NAPEs according to the invention, analyzed in the AD population compared with the controls; significance levels in an unpaired 2-tailed t-test (control vs AD) and AUC values of the corresponding ROC curve.**

| **pNAPE** | **% difference** | **P value** | **Significance** | **AUC** |
|---|---|---|---|---|
| 1_P16:0-18:1-N16:0 | -11% | 0.0002 | *** | 0.689 |
| 2_P18:0-18:2-N16:0 | 9% | 0.0046 | ** | 0.644 |
| 4_P16:0-22:5-N16:0 | -45% | <0.0001 | *** | 0.912 |
| 5_P16:0-22:4-N16:0 | -5% | 0.0106 | * | 0.603 |
| 7_P16:0-22:5-N18:1 | -59% | <0.0001 | *** | 0.918 |
| 10_P18:0-20:4-N18:1 | -8% | <0.0001 | *** | 0.694 |
| 11 _P18:0-20:5-N18:0 | 5% | 0.0242 | * | 0.615 |
| 12_P18:0-22:4-N16:0 | 13% | 0.0001 | *** | 0.696 |
| 13_P16:0-22:4-N18:0 | 23% | <0.0001 | *** | 0.833 |
| 15_P18:0-22:6-N18:1 | -5% | 0.1754 | ns | 0.569 |
| 17_P18:0-22:4-N18:1 | 13% | 0.0003 | *** | 0.682 |
| 18_P18:0-22:4-N18:0 | 19% | <0.0001 | *** | 0.716 |

The two populations analyzed differ significantly. The data regarding the AD patients were obtained, as shown previously (WO2019/091952), from a sample of 30 individuals resident in Italy, while the data obtained with the NAPEs described herein relate to a cohort of 65 individuals resident in Austria. The different numbers and geographical origin (and therefore the type of diet) of the patients may explain the differences observed in the NAPE levels.

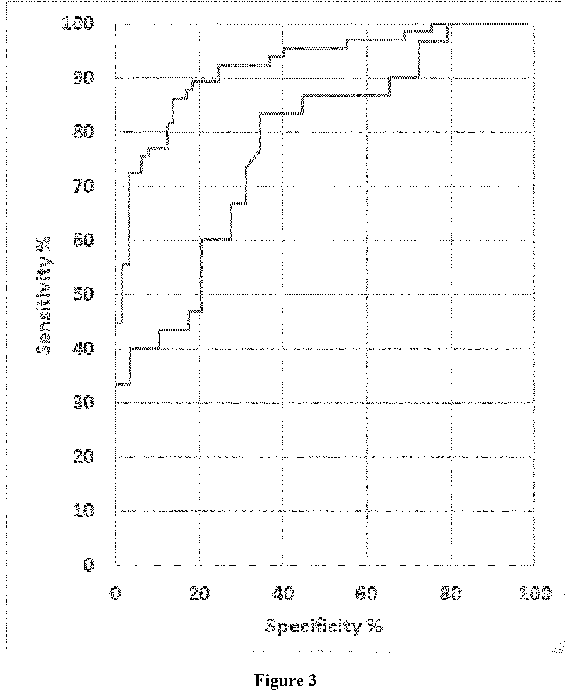

The two best ROC curves obtained with the preceding biomarkers were then compared with those obtained according to the present invention. Three NAPEs from the preceding group of biomarkers exhibited very similar AUC values (Table 2). The highest AUC value for NAPE P16:0-22:5-N18:1, as predictor of AD, was obtained from the ROC curves of the novel biomarkers. Figure 3 shows the superimposed curves of P18:0-22:-N18:0, the best marker according to the prior art, and P16:0-22:5-N18:1, the best marker according to the present invention (top curve).

Analysis of the 5% and 95% quantiles of the distribution of each NAPE in the healthy population demonstrates that plasma levels of NAPE lower or higher than the reference values set out in **Table 3** below are indicative of a potential state of AD:

**Table 3. Threshold values of NAPE concentration (in pmol/ml) indicative of an AD state. The data are calculated on the basis of the 5% and 95% quantiles observed in the cognitively healthy population.**

| **NAPE** | **Values indicative of AD** | **Reference value pmol/ml** |
|---|---|---|
| 1_P16:0-18:1-N16:0 | Above | 30.1 |
| 2_P18:0-18:2-N16:0 | Below | 40.4 |
| 4_P16:0-22:5-N16:0 | Above | 84.4 |
| 5_P16:0-22:4-N16:0 | Above | 190.0 |
| 7_P16:0-22:5-N18:1 | Above | 33.2 |
| 10 _P18:0-20:4-N18: | Above | 74.9 |
| 11_P18:0-20:5-N18: | Below | 31.6 |
| 12_P18:0-22:4-N16: | Below | 21.8 |
| 13_P16:0-22:4-N18: | Below | 49.9 |
| 15_P18:0-22:6-N18: | Above | 19.2 |
| 17_P18:0-22:4-N18: | Below | 7.5 |
| 18_P18:0-22:4-N18: | Below | 7.1 |

To determine said reference values, the distribution of the levels of each NAPE in 65 individuals of the control cohort was analyzed, assuming that said levels represent the normal concentration range of said lipids in the plasma of a generic cognitively healthy population. The mean of the corresponding NAPEs in the AD cohort was than calculated, and compared with the 5% or 95% quantile of the control cohort. The reference values for AD were set by assuming a 5% risk of error, and determined below the 5% quantile for the NAPEs which exhibited a tendency to decrease in the AD group, and above the 95% quantile for the NAPEs which exhibited a tendency to increase in the AD population.

Compared with the earlier data (WO2019/091952), the group of AD biomarkers according to the present invention is clearly far more specific and sensitive. The best AUC curve obtained with the earlier biomarkers on 30 individuals (Italian origin) amounted to 0.78, whereas the AUC curve obtained with NAPE biomarkers P16:0-22:5-N18:1 according to the present invention (patients' origin: Austria) amounts to 0.92, and is therefore significantly greater. Using the combination of biomarkers according to the present invention, the total predictive power can be increased to 0.95.

### Example 3

### Diagnostic kit

The kit contains:
1) detailed description of the entire analysis procedure;
2) detailed description of the automation protocol, comprising step-by-step instructions for the automated station if the user laboratory has one;
3) pre-weighed vials of very high purity ammonium formate, ready to be dissolved in water and very high quality acetonitrile (LC-MS grade) in the laboratory at which the analysis is conducted;
4) pre-weighed vials containing exogenous NAPE produced by synthesis (18:1/18:1/N19:0), which is useful to prepare the calibration curve used to quantify the NAPEs;
5) pre-aliquoted vials of commercial human plasma, obtained from the population NOT forming the subject of the study, which are useful to prepare the calibration curve;
6) aliquots of pre-aliquoted vials of murine plasma, useful to prepare the QC samples.

## Claims

1. An *in vitro* method for determining the presence or the risk of onset of Alzheimer's disease, for monitoring its progression and/or for determining the efficacy of a pharmacological treatment thereof in a subject, said method comprising:
a) determining the concentration of each one of the following N-acylphosphatidylethanolamines (NAPEs) in a plasma sample from the subject:
| | | |
|---|---|---|
| P16:0/18:1/N16:0; | P18:0/18:2/N16:0; | P16:0/22:5/N16:0; |
| P16:0/22:4/N16:0; | P16:0/22:5/N18:1; | P18:0/20:4/N18:1; |
| P18:0/20:5/N18:0; | P18:0/22:4/N16:0; | P16:0/22:4/N18:0; |
| P18:0/22:6/N18:1; | P18:0/22:4/N18:1; | P18:0/22:4/N18:0; |
b) comparing the concentration of each NAPE with a reference value corresponding to the same NAPE concentration in the plasma of subjects not affected by Alzheimer's disease,
wherein a difference between each NAPE concentration detected in the plasma sample of the test subject and the corresponding reference value indicates the presence or risk of onset of Alzheimer's disease, its progression or the inefficacy of the pharmacological treatment.

2. Method according to claim 1, wherein the NAPE concentration in the plasma sample is determined by liquid chromatography coupled to tandem mass spectrometry (LC-MS/MS).

3. Method according to claim 2, wherein said LC-MS/MS analysis is performed with an automated instrument able to analyze multiple samples in multiwell plates.

4. Method according to claims 1-3, wherein the NAPE species present in the plasma sample are quantitated using a calibration curve prepared by adding a synthetic NAPE at increasing concentrations to commercial human plasma.

5. Method according to claim 1, wherein said difference compared with the reference value corresponds to:
a) an increased concentration of the following NAPEs: P16:0/18:1/N16:0; P16:0/22:5/N16:0; P16:0/22:4/N16:0; P16:0/22:5/N18:1; P18:0/20:4/N18:1; P18:0/22:6/N18:1;
b) a decreased concentration of the following NAPEs: P18:0/18:2/N16:0; P18:0/20:5/N18:0; P18:0/22:4/N16:0; P16:0/22:4/N18:0 P18:0/22:4/N18:1; P18:0/22:4/N18:0.

6. Method according to claim 5, wherein said concentration increase or decrease, compared with the reference value, is at least 5%, preferably at least 10%, more preferably at least 30%, yet more preferably at least 60%.

7. Method according to claims 5-6, wherein said reference value concentration for each NAPE is the following, expressed in pmol/ml:
| | |
|---|---|
| P16:0/18:1/N16:0 | 30.1 |
| P18:0/18:2/N16:0 | 40.4 |
| P16:0/22:5/N16:0 | 84.4 |
| P16:0/22:4/N16:0 | 190.0 |
| P16:0/22:5/N18:1 | 33.2 |
| P18:0/20:4/N18:1 | 74.9 |
| P18:0/20:5/N18:0 | 31.6 |
| P18:0/22:4/N16:0 | 21.8 |
| P16:0/22:4/N18:0 | 49.9 |
| P18:0/22:6/N18:1 | 19.2 |
| P18:0/22:4/N18:1 | 7.5 |
| P18:0/22:4/N18:0 | 7.1. |

8. Kit for implementing a method according to any one of the preceding claims, said kit comprising known amounts of all the NAPE species disclosed in claim 1, as calibration standards.

9. Kit according to claim 8, further comprising reagents and buffers for preparing the sample to be analyzed and performing the analysis, particularly by the LC-MS/MS technique.

## Patentansprüche

1. *In-vitro*-Verfahren zur Bestimmung des Vorliegens oder des Risikos des Ausbruchs der Alzheimer-Krankheit, zur Überwachung ihres Fortschreitens und/oder zur Bestimmung der Wirksamkeit einer pharmakologischen Behandlung davon bei einem Individuum, wobei das Verfahren Folgendes umfasst:
a) Bestimmen der Konzentration von jedem der folgenden N-Acylphosphatidylethanolamine (NAPEs) in einer Plasmaprobe von dem Individuum:
| | | |
|---|---|---|
| P16:0/18:1/N16:0; | P18:0/18:2/N16:0; | P16:0/22:5/N16:0; |
| P16:0/22:4/N16:0; | P16:0/22:5/N18:1; | P18:0/20:4/N18:1; |
| P18:0/20:5/N18:0; | P18:0/22:4/N16:0; | P16:0/22:4/N18:0; |
| P18:0/22:6/N18:1; | P18:0/22:4/N18:1; | P18:0/22:4/N18:0; |
b) Vergleichen der Konzentration jedes NAPE mit einem Referenzwert, der der gleichen NAPE-Konzentration im Plasma von Individuen entspricht, die nicht von der Alzheimer-Krankheit betroffen sind,
wobei eine Differenz zwischen jeder NAPE-Konzentration, die in der Plasmaprobe des Testindividuums nachgewiesen wird, und dem entsprechenden Referenzwert das Vorliegen oder das Risiko des Ausbruchs der Alzheimer-Krankheit, ihr Fortschreiten oder die Unwirksamkeit der pharmakologischen Behandlung anzeigt.

2. Verfahren nach Anspruch 1, wobei die NAPE-Konzentration in der Plasmaprobe durch Flüssigchromatographie in Verbindung mit Tandem-Massenspektrometrie (LC-MS/MS) bestimmt wird.

3. Verfahren nach Anspruch 2, wobei die LC-MS/MS-Analyse mit einem automatisierten Instrument durchgeführt wird, das in der Lage ist, mehrere Proben in Multiwell-Platten zu analysieren.

4. Verfahren nach den Ansprüchen 1-3, wobei die NAPE-Spezies, die in der Plasmaprobe vorhanden sind, unter Verwendung einer Kalibrierungskurve quantifiziert werden, die durch Zugabe eines synthetischen NAPE in zunehmenden Konzentrationen zu kommerziellem menschlichem Plasma hergestellt wird.

5. Verfahren nach Anspruch 1, wobei die Differenz verglichen mit dem Referenzwert Folgendem entspricht:
a) einer erhöhten Konzentration der folgenden NAPEs: P16:0/18:1/N16:0; P16:0/22:5/N16:0; P16:0/22:4/N16:0; P16:0/22:5/N18:1; P18:0/20:4/N18:1; P18:0/22:6/N18:1;
b) einer verringerten Konzentration der folgenden NAPEs: P18:0/18:2/N16:0; P18:0/20:5/N18:0; P18:0/22:4/N16:0; P16:0/22:4/N18:0; P18:0/22:4/N18:1; P18:0/22:4/N18:0.

6. Verfahren nach Anspruch 5, wobei die Konzentrationserhöhung oder - verringerung, verglichen mit dem Referenzwert, mindestens 5%, vorzugsweise mindestens 10%, mehr bevorzugt mindestens 30%, noch mehr bevorzugt mindestens 60% beträgt.

7. Verfahren nach den Ansprüchen 5-6, wobei die Referenzwertkonzentration für jedes NAPE die folgende ist, ausgedrückt in pmol/ml:
| | |
|---|---|
| P16:0/18:1/N16:0 | 30,1 |
| P18:0/18:2/N16:0 | 40,4 |
| P16:0/22:5/N16:0 | 84,4 |
| P16:0/22:4/N16:0 | 190,0 |
| P16:0/22:5/N18: 1 | 33,2 |
| P18:0/20:4/N18: 1 | 74,9 |
| P18:0/20:5/N18:0 | 31,6 |
| P18:0/22:4/N16:0 | 21,8 |
| P16:0/22:4/N18:0 | 49,9 |
| P18:0/22:6/N18:1 | 19,2 |
| P18:0/22:4/N18: 1 | 7,5 |
| P18:0/22:4/N18:0 | 7,1. |

8. Kit zur Implementierung eines Verfahrens nach einem der vorhergehenden Ansprüche, wobei das Kit bekannte Mengen aller NAPE-Spezies, die in Anspruch 1 offenbart sind, als Kalibrierungsstandards umfasst.

9. Kit nach Anspruch 8, ferner umfassend Reagenzien und Puffer zur Herstellung der zu analysierenden Probe und zur Durchführung der Analyse, insbesondere durch die LC-MS/MS-Technik.

## Revendications

1. Procédé *in vitro* destiné à déterminer la présence ou le risque d'apparition de la maladie d'Alzheimer, à surveiller la progression de celle-ci et/ou à déterminer l'efficacité d'un traitement pharmacologique de cette dernière chez un sujet, ledit procédé comprenant le fait de :
a) déterminer la concentration de chacune des N-acylphosphatidyl-éthanolamines (NAPE) suivantes dans un échantillon de plasma du sujet :
P16:0/18:1/N16:0 ; P18:0/18:2/N16:0 ; P16:0/22:5/N16:0 ;
P16:0/22:4/N16:0 ; P16:0/22:5/N18:1 ; P18:0/20:4/N18:1 ;
P18:0/20:5/N18:0 ; P18:0/22:4/N16:0 ; P16:0/22:4/N18:0 ;
P18:0/22:6/N18:1 ; P18:0/22:4/N18:1 ; P18:0/22:4/N18:0 ;
b) comparer la concentration de chaque NAPE à une valeur de référence qui correspond à la même concentration de NAPE dans le plasma de sujets qui ne sont pas affectés par la maladie d'Alzheimer ;
dans lequel une différence entre chaque concentration de NAPE détectée dans l'échantillon de plasma du sujet soumis au test et la valeur de référence correspondante témoigne de la présence ou du risque d'apparition de la maladie d'Alzheimer, de sa progression ou de l'inefficacité du traitement pharmacologique.

2. Procédé selon la revendication 1, dans lequel la concentration des NAPE dans l'échantillon de plasma est déterminée par une chromatographie en phase liquide couplée à une spectrométrie de masse en tandem (LC-MS/MS).

3. Procédé selon la revendication 2, dans lequel ladite analyse LC-MS/MS est mise en œuvre avec un instrument automatisé capable d'analyser de multiples échantillons dans des plaques multipuits.

4. Procédé selon les revendications 1 à 3, dans lequel les espèces NAPE présentes dans l'échantillon de plasma sont quantifiées en utilisant une courbe d'étalonnage que l'on prépare en ajoutant une NAPE de synthèse à des concentrations croissantes à du plasma humain disponible dans le commerce.

5. Procédé selon la revendication 1, dans lequel ladite différence comparée à la valeur de référence correspond à :
a) une concentration accrue des NAPE suivantes : P16:0/18:1/N16:0 ; P16:0/22:5/N16:0 ; P16:0/22:4/N16:0 ; P16:0/22:5/N18:1 ; P18:0/20:4/N18:1 ; P18:0/22:6/N18:1 ;
b) une concentration réduite des NAPE suivantes : P18:0/18:2/N16:0 ; P18:0/20:5/N18:0 ; P18:0/22:4/N16:0 ; P16:0/22:4/N18:0 ; P18:0/22:4/N18:1 ; P18:0/22:4/N18:0.

6. Procédé selon la revendication 5, dans lequel ladite augmentation ou diminution de la concentration, comparée à la valeur de référence, s'élève à au moins 5 %, de préférence à au moins 10 %, de manière plus préférée à au moins 30 %, de manière encore plus préférée à au moins 60 %.

7. Procédé selon les revendications 5 à 6, dans lequel ladite concentration faisant office de valeur de référence pour chaque NAPE est la suivante, exprimée en pmol/ml :
P16:0/18:1/N16:0 30,1
P18:0/18:2/N16:0 40,4
P16:0/22:5/N16:0 84,4
P16:0/22:4/N16:0 190,0
P16:0/22:5/N18:1 33,2
P18:0/20:4/N18:1 74,9
P18:0/20:5/N18:0 31,6
P18:0/22:4/N16:0 21,8
P16:0/22:4/N18:0 49,9
P18:0/22:6/N18:1 19,2
P18:0/22:4/N18:1 7,5
P18:0/22:4/N18:0 7,1.

8. Kit destiné à la mise en œuvre d'un procédé selon l'une quelconque des revendications précédentes, ledit kit comprend des quantités connues de toutes les espèces NAPE révélées dans la revendication 1, à titre de normes d'étalonnage.

9. Kit selon la revendication 8, qui comprend en outre des réactifs et des tampons pour la préparation de l'échantillon à analyser et pour la mise en œuvre de l'analyse, en particulier par la technique LC-MS/MS.
